# EUROPEAN PATENT SPECIFICATION

(11) **EP 0 558 130 B1**
(45) Date of publication and mention of the grant of the patent: **15.04.1998**
(21) Application number: 93200473.2
(22) Date of filing: 19.02.1993
(51) Int. Cl.: G01N 25/00, G01N 25/14, G01N 25/16, C09C 1/48, C01B 31/20, G01N 33/22

(54) **Apparatus for analysing carbon products**
Vorrichtung zur Analyse von Kohlenstoffprodukten
Appareil d'analyse de produits de charbon

(30) Priority: 28.02.1992 NO 920790
(43) Date of publication of application: 01.09.1993
(73) Proprietor: NORSK HYDRO ASA, 0240 Oslo (NO)
(72) Inventor: Bergli, Knut, N-3472 Bodalen (NO); Foosnaes, Trygve, N-5875 Ardalstangen (NO); Naterstad, Tormod, N-1370 Asker (NO)
(74) Representative: Bleukx, Luc

(56) References cited:
- GB-A- 2 154 318
- US-A- 4 976 549
- PATENT ABSTRACTS OF JAPAN vol. 10, no. 22 (P-424) 28 January 1986 & JP-A-60 174 951 (SUMITOMO KINZOKU KOGYO)

## Description

The present invention concerns apparatus for the analysis of granular coke, green and burned carbon core samples.

It is the desire of every metal producer to have as much knowledge as possible about the properties of carbon-resistant parts of production equipment, such as anodes, cathodes and ramming paste.
There are many factors which decide how a carbon product will react during the metal production process.

When the carbon product comes into contact with the air, in the way that the upper part of the anode usually does, the carbon will corrode. Another unfortunate process which mainly occurs on the underside of, for example, the anodes, is that some of the CO₂ gas from the primary reaction reacts with the carbon to form carbon monoxide. These two conditions are called air reactivity and CO₂ reactivity, respectively.

The reaction with the air and CO₂ can lead to crumbling of the anode material which often has the result that operational problems occur with anode particles in the electrolyte. This is called sooting.

The coefficient of thermal expansion (CTE) is a property of a carbon product which it is useful to know in metal production. Analysis of the coefficient of thermal expansion is called dilatometry.

GB-A-2 154 318, discloses a method for assessing the micro-reactivity of coke, more specific the CO₂-reactivity of such material. This publication further discloses that the equipment involved may comprise a furnace, a device for weighing, a device for temperature registration and a device for the introduction of gas in the furnace.

US 4, 976, 549 relates to a microdilameter for direct measurement of coal ash sintering and fusing properties at elevated temperatures and pressures. The equipment comprises electrical resistivity measurements across a sample to provide a measurement of the onset of the sintering and fusion of the ash particulates while the contraction of the sample during sintering is measured with a linear variable displacement transducer for detecting the initiation of sintering.

However, this state-of-the-art equipment are only able to decide either the reactivity or the coefficient of thermal expansion. In addition, the existing equipment are often large and imprecise and complicated and time-consuming to use.

There has, therefore, long been a need for a more practical apparatus to measure these parameters in carbon products.

The present invention concerns an apparatus for the analysis of carbon products with regard to air reactivity, CO₂ reactivity, the soot index and the coefficient of thermal expansion. All these properties of a carbon product can be determined by using the apparatus in the present invention.

This apparatus thus has a wide range of advantages compared with what was previously known in this field. Now only one apparatus is required to analyse a carbon product with regard to the above properties, whereas previously it was necessary to use at least two different apparatuses. This represents both a cost saving and a time saving and also a saving in the number of operators required to operate the apparatus. In addition, the apparatus in accordance with the present invention has a compact design which means that it requires less room.

The apparatus comprises, among other things, a processing unit which controls the various instruments and registers and processes the analytical data. This has the result that the analyses which are carried out on this apparatus are very precise and rapid. For this reason, the apparatus is also easy to operate and, by the fact that the processing unit carries out the necessary process control, the apparatus is both environmentally friendly and user friendly.

The apparatus for the analysis of carbon products in accordance with the present invention is characterised by the features of claim 1.

The carbon samples which are to be analysed for their reactivity and soot index are suspended via a sample holder in a weighing device, while the carbon samples which are to be analysed for their coefficient of thermal expansion are connected to a differential transformer via a sample holder.

The reactivity and soot index analyses can be carried out on granular coke and burned carbon core samples. Dilatometry can be carried out on green and burned carbon core samples.

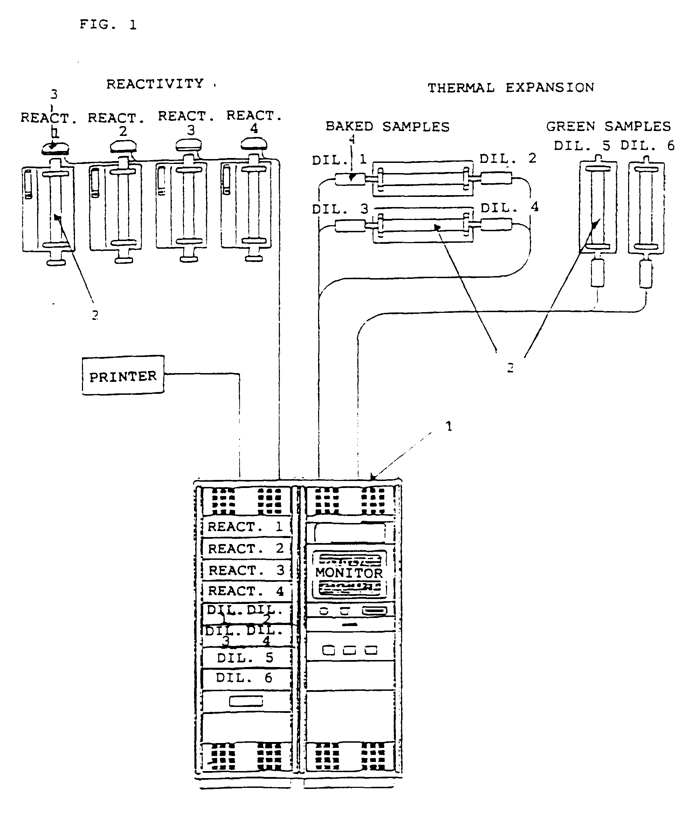

The apparatus according to the present invention is shown in figure 1, where the symbols 1-4 stand for:

| | |
|---|---|
| 1. | Processing unit |
| 2. | Tube furnace |
| 3. | Weighing device |
| 4. | Dilatometer |

In principle there is no limit to how many carbon samples can be analysed in parallel by connecting several tube furnaces to the computer equipment.

When determining air reactivity, CO₂ reactivity and the soot index, vertical tube furnaces with inlets for the introduction of gas are used, whereby it is the sample holder for the carbon product which is freely suspended from a weighing device and which reaches down into the tube furnace; the sample holder is provided with one or more thermocouples for registering the temperature in the carbon product.

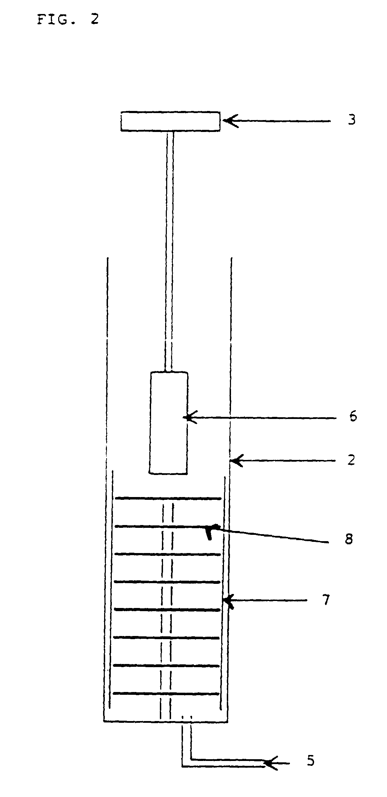

This apparatus is shown in figure 2 and the symbols 2, 3 and 5-8 stand for:

| | |
|---|---|
| 2. | Tube furnace |
| 3. | Weighing device |
| 5. | Inlet for the introduction of gas |
| 6. | Sample holder |
| 7. | Radial radiation shield |
| 8. | Heating element |

For dilatometry vertical tube furnaces are used for green samples, whereas horizontal furnaces are used for burned samples. Green samples must stand vertically during the analysis because the relatively soft material in these samples will fasten itself to the sample holder and give incorrect results if it is horizontal. In the horizontal tube furnaces two burned samples can be analysed simultaneously in one furnace by placing one sample at each end of the furnace.

These tube furnaces with inlets for the introduction of gas form dilatometers consisting of a sample holder for the carbon product which is placed a little way into the furnace; the sample holder, which is provided with a thermocouple, is connected to a differential transformer at the end of the tube furnace.

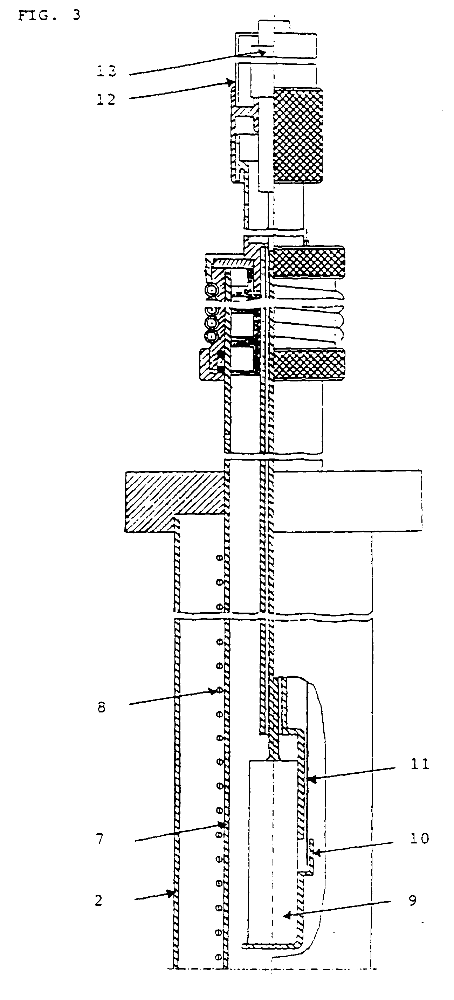

Figure 3 shows a sketch of this device and symbols 2 and 7-13 stand for:

| | |
|---|---|
| 2. | Tube furnace |
| 7. | Radial radiation shield |
| 8. | Heating element |
| 9. | Carbon sample |
| 10. | Sample holder |
| 11. | Thermocouple |
| 12. | Housing for differential transformer |
| 13. | Differential transformer |

This apparatus for the analysis of carbon products is applicable both for research and for routine tests in anode production.

The invention will be described further in the following.

### Analysis of reactivity and soot index

Analysis of reactivity and the soot index of carbon samples is carried out at constant temperatures. The weight loss of the carbon sample, which is due to gasification by air and carbon dioxide, is measured continuously. In an equilibrium reaction the loss of weight is proportional to time. The loss of weight as a function of time is an expression of the reaction speed which is thus called reactivity (air or CO₂ reactivity). The quantity of soot dust which is generated during the analysis is collected and weighed and provides the basis for the calculation of a dimensionless parameter, namely the soot index.

The carbon sample is suspended by means of the sample holder 6 a little way down into the vertical tube furnace 2 which is made of gold. In order that the weight loss may be followed continuously by the processing unit 1, the sample is suspended via the holder 6 from the weighing device 3. A thermocouple is also connected to the sample so that the temperature in the sample can be registered and checked. The temperature in the furnace and the sample are regulated by the processing unit.

The sample holders 6 used in the analysis of granular coke and burned carbon core samples are shaped differently, but both are shaped so that the thermocouple is in contact with the carbon product itself when the temperature is registered. This means that the temperature registration is very precise.

The gas is introduced into the furnace 2 from an inlet 5 in the base and is preheated to the reaction temperature as it passes a radial radiation shield 7 within the furnace on the way towards the carbon sample. The introduction of the gas is also regulated by the processing unit 1.

The analysis of the carbon product for reactivity and the soot index is carried out automatically by the processing unit 1 via dialogue boxes. The processing unit switches from the introduction of one gas to the other automatically. During the heating of the sample inert atmosphere (N₂) is introduced. The processing unit closes the N₂ valve automatically and opens the air or CO₂ valve. When the reaction has been completed the processing unit switches automatically back to N₂ and the sample is cooled down.
Standard conditions during this analysis are:

| | |
|---|---|
| Heating time | 60 minutes |
| Reaction time | 180 minutes |
| Cooling time | 30 minutes |

| | |
|---|---|
| Reaction temperature in CO₂ | 960°C |
| Reaction temperature in air | 525°C |

The flow of gas through the furnace is 100 Nl/h of CO₂ and 200 Nl/h of air.

These reaction conditions may, however, be changed easily by the operator.

The weighing system in the apparatus has a reproducibility of 1 mg. The weight is registered on a continuous basis (every 20 seconds according to standard conditions). The high number of measurements, the good reproducibility of the weighing system and the advanced temperature control, which is within ± 1° of the desired temperature, ensure high precision results. The precision is better than ± 1 %.

The results of the analysis regarding the reactivities and the soot index are calculated by the processing unit 1.

In an apparatus which consists of 8 tube furnaces, it is possible to analyse 8 carbon samples in the course of 4.5 hours. The time required to prepare a carbon sample for analysis is 10 minutes. As mentioned above, the processing unit controls the furnaces automatically. The time required for an operator to be able to prepare the samples, fasten the samples in the furnaces, remove the samples from the furnaces, collect the soot and read off the results for samples in 8 furnaces is a total of 100 minutes.

### Dilatometry

When the apparatus is used as a dilatometer, the weighing device 3, to which the carbon sample is attached during the reactivity analysis, is replaced by a differential transformer 13. In addition, another sample holder 10 is used, provided with a thermocouple 11 and connected to the differential transformer 13. This differential transformer 13 makes it possible to register the volume changes and temperature of the sample in the processing unit 1, and the coefficient of thermal expansion can be calculated.

The coefficient of thermal expansion (CTE) is expressed as the average increase in a graph of the carbon sample's expansion as a function of the temperature.

Analysis of the carbon product regarding the coefficient of thermal expansion is carried out by first introducing the N₂ gas. The carbon product is heated up from room temperature to 800°C at a standard heating rate of 10° per minute. The heating conditions can easily be changed by the operator if desired. The temperature and expansion are registered and plotted against one another. When the measurements have been completed the furnace is cooled down to room temperature. During the cooling from the maximum temperature to 400°C, the cooling speed is increased by adding N₂ under high pressure. When the temperature has dropped below 400°C air under high pressure is used as a cooling medium. The whole of this process occurs automatically under the control of the processing unit 1.

The coefficient of thermal expansion is calculated by the processing unit 1 on the basis of data which are stored during the dilatometry.

This analysis lasts for 80 minutes plus the cooling time. The time which is required for an operator to prepare the samples, fasten the samples in the furnaces, remove the samples from the furnaces and read off the results for the samples on 10 dilatometers is a total of 120 minutes.

## Claims

1. Apparatus for determining the air reactivity, CO₂ reactivity, soot index, and coefficient of thermal expansion of a carbon product, including:
at least one tube furnace for determining the air reactivity, CO₂ reactivity and soot index of the carbon product including, an inlet for the introduction of gas to the furnace (2), and a sample holder (6) provided with a device for measuring temperature and a device for weighing (3); and at least one tube furnace (2) for determining the coefficient of thermal expansion of the carbon product, including an inlet for the introduction of gas, and a sample holder provided with a device for measuring temperature and a device for registration of dilation (4); and a joint processing unit (1) connected to each said furnace (2) which processes the analytical data produced by the various instruments.

2. An apparatus according to claim 1, wherein said least one tube furnace for determining the air reactivity, CO₂ reactivity, and soot index of the carbon product, has said sample holder (6) freely suspended from said weighing device (3) and reaching down into the tube furnace (2), and said temperature measuring device comprises one or more thermocouples on the sample holder (6).

3. Apparatus according to claim 1 or claim 2, wherein said at least one tube furnace (2) for determining the coefficient of thermal expansion of the carbon product, has said sample holder which is placed a little way into the furnace (2) and forming at least part of a dilatometer, the sample holder being provided with a thermocouple (11) and being connected to a differential transformer (13) in the end of the furnace (2).

## Patentansprüche

1. Zur Bestimmung der Reaktivität von Luft, der Reaktivität von CO₂, des Rußindexes und des Koeffizienten der thermischen Expansion eines Kohlenstoffproduktes dienende Vorrichtung mit:
wenigstens einem zur Bestimmung der Reaktivität von Luft, der Reaktivität von CO₂ und des Rußindexes des Kohlenstoffproduktes dienenden Röhrenofen, der eine Einlaßöffnung für die Einleitung von Gas in den Ofen (2) und einen Probehalter (6) aufweist, welch letzterer mit einem Gerät für die Temperaturmessung und mit einer Wägevorrichtung ausgestattet ist,
mit wenigstens einem zur Bestimmung des Koeffizienten der thermischen Expansion des Kohlenstoffproduktes dienenden Röhrenofen (2), der eine Einlaßöffnung für die Einleitung von Gas und einen Probehalter (6) aufweist, welch letzterer mit einem Gerät für die Temperaturmessung und mit einem Gerät zur Aufzeichnung der Dehnung (4) ausgestattet ist,
und mit einer gemeinsamen Datenverarbeitungseinheit (1), welche an einen jeden Ofen (2) angeschlossen ist und welche die von den verschiedenen Instrumenten erzeugten analytischen Daten verarbeitet.

2. Vorrichtung nach Anspruch 1, gemäß welcher wenigstens ein zur Bestimmung der Reaktivität von Luft, der Reaktivität von CO₂ und des Rußindexes des Kohlenstoffproduktes dienender Röhrenofen mit dem Probehalter (6) ausgestattet ist, welch letzterer freischwebend an der Wägevorrichtung (3) aufgehängt ist und herunter bis in den Röhrenofen(2) hineinreicht; und das Gerät für die Temperaturmessung weist ein oder mehrere Thermoelemente an dem Probehalter (6) auf.

3. Vorrichtung nach Anspruch 1 und Anspruch 2, gemäß welcher wenigstens ein zur Bestimmung des Koeffizienten der thermischen Expansion des Kohlenstoffproduktes dienender Röhrenofen (2) mit dem Probehalter ausgestattet ist, welch letzterer eine kleines Stück in den Ofen (2) hineingeführt ist und wenigstens einen Teil eines Dilatometers bildet, wobei der Probehalter mit einem Thermoelement (11) ausgestattet ist und an einen Differentialtransformator (13) am Ende des Ofens (2) angeschlossen ist.

## Revendications

1. Appareil pour déterminer la réactivité d'air, la réactivité de CO₂, l'indice de suie ainsi que le coefficient d'expansion thermique d'un produit de carbone, incluant:
au moins un four tubulaire pour la détermination de la réactivité d'air, de la réactivité de CO₂, ainsi que de l'indice de suie du produit de carbone, comportant une admission pour l'introduction de gaz dans le four (2) et un porte-échantillon (6) pourvu d'un dispositif pour mesurer la température ainsi que d'un dispositif de pesée (3),
et au moins un four tubulaire (2) pour la détermination du coefficient d'expansion thermique du produit de carbone, comportant une admission pour l'introduction de gaz et un porte-échantillon pourvu d'un dispositif pour mesurer la température ainsi que d'un dispositif pour l'enregistrement de la dilatation (4),
ainsi qu'une unité de traitement commune (1), reliée à chacun desdits fours, qui traite les données analytiques produites par les divers instruments.

2. Appareil conforme à la revendication 1, suivant lequel ledit four, au nombre d'au moins un, servant à la détermination de la réactivité d'air, de la réactivité de CO₂ et de l'indice de suie du produit de carbone comporte ledit porte-échantillon (6) sous forme librement suspendue à partir dudit dispositif de pesée (3) et s'étendant vers le bas dans le four tubulaire (2), et ledit dispositif pour mesurer la température comporte un ou plusieurs thermocouples sur le porte-échantillon (6).

3. Appareil conforme à la revendication 1 ou à la revendication 2, suivant lequel ledit four (2), au nombre d'au moins un, servant à la détermination du coefficient de dilatation thermique du produit de carbone est pourvu dudit porte-échantillon qui est disposé un bout de chemin à l'intérieur du four (2) et forme au moins une partie d'un dilatomètre, le porte-échantillon étant pourvu d'un thermocouple (11) et étant relié à un transformateur différentiel (13) à l'extrémité du four (2).
